# EUROPEAN PATENT SPECIFICATION

(11) **EP 4 149 691 B1**
(45) Date of publication and mention of the grant of the patent: **14.08.2024**
(21) Application number: 21725099.2
(22) Date of filing: 07.05.2021
(51) Int. Cl.: B06B 1/02, A61B 8/00

(54) **AN ULTRASOUND TRANSDUCER AND A TILED ARRAY OF ULTRASOUND TRANSDUCERS**
ULTRASCHALLWANDLER UND GEKACHELTE ANORDNUNG VON ULTRASCHALLWANDLERN
TRANSDUCTEUR À ULTRASONS ET RÉSEAU EN MOSAÏQUE DE TRANSDUCTEURS À ULTRASONS

(30) Priority: 14.05.2020 EP 20174763
(43) Date of publication of application: 22.03.2023
(73) Proprietor: Koninklijke Philips N.V., 5656 AG Eindhoven (NL)
(72) Inventor: VAN RENS, Antonia, Cornelia, 5656 AE Eindhoven (NL); HENNEKEN, Vincent, Adrianus, 5656 AE Eindhoven (NL); KLOOTWIJK, Johan, Hendrik, 5656 AE Eindhoven (NL)
(74) Representative: Philips Intellectual Property & Standards
(86) International application number: PCT/EP2021/062184
(87) International publication number: WO 2021/228720

(56) References cited:
- US-A1- 2005 094 490
- US-A1- 2016 136 686
- US-A1- 2018 029 077
- US-A1- 2020 066 966

## Description

### FIELD OF THE INVENTION

The invention relates to ultrasound transducers, in particular having a two dimensional array of transducer elements provided over associated control circuitry.

### BACKGROUND OF THE INVENTION

Two dimensional arrays of ultrasound transducer elements are well known, for example for use in 3D ultrasound probes. Such probes integrate a 2-dimensional matrix array of transducer elements with a controlling ASIC. The ASIC contains circuitry to control, transmit, and receive acoustic signals of individual transducer elements.

The required functionality of the ASIC is laid out in unit control cells that are pitch matched to the transducer elements. The size of the transducer elements is determined by the imaging requirements of the application; specifically the frequency and field of view.

A typical size of the transducer elements in a 2D array is between 100 and 350µm. The size may slightly differ in the horizontal and vertical direction. A typical matrix array contains between 1000 and 10,000 transducer elements.

Such an array and associated single ASIC circuitry is termed "an ultrasound transducer" in this document. An individual sensing element is termed "an ultrasound transducer element". A large tiled or stitched array of multiple such ultrasound transducers is termed "an ultrasound transducer arrangement".

Conventionally, matrix arrays have been built using piezoelectric materials for the transducer elements. Nowadays, lower cost transducer arrays are feasible using capacitive micro-machined ultrasound transducers (cMUTs) which can be manufactured using standard semiconductor processing techniques. Depending on the chosen fabrication route, these transducer elements can be integrated monolithically on the controlling ASIC.

Depending on the type of probe, the size and aspect ratio of the transducer aperture can vary considerably; for example a phased array cardiac probe may have an aspect ratio of 1.5 (2 x 1.3 cm) and a linear transducer probe may have an aspect ratio of 12 (6 x 0.5 cm). In most cases, the width of the probe aperture is smaller than 2 cm.

Realization of 2D ultrasound transducer arrangements with large dimensions imposes additional design challenges as the dimensions exceed the useful area of a reticle of state-of-the-art lithographic equipment (steppers / scanners). A typical useful area is about 2.5x2.5 cm. To realize larger transducer arrays, a form of array stitching is needed or transducer arrays need to be tiled as mentioned above.

For image quality, it is important that the transducer array is homogeneous. This means that tiled or stitched solutions should use modules originating from the same wafer; preferably from neighboring positions. In addition, gaps between transducer modules should be small for example < 0.3 λ and gap sizes should be known to be able to (partly) compensate for the effects of any such gap on the beamforming process.

Generally, the most expensive and most complex part of a 2-dimensional (matrix) array of cMUT transducer elements with a monolithically integrated controlling ASIC is the underlying ASIC functionality. ASIC (re)design is extremely costly and time consuming, and IC mask set costs for advanced technologies can be in the range of a few hundred thousand dollars to a million dollars. Therefore, it is very attractive to be able to reuse ASIC designs for varying transducer designs in terms of center frequencies and/or transducer apertures.

In conventional transducer designs this is hardly possible, as the ASIC unit control cells are pitch-matched to the transducer elements, allowing no flexibility in placing the transducer elements at different positions relative to the underlying ASIC unit control cells.

It is also difficult to tile modules that are realized in CMOS IC foundries in a quasi-seamless way. An important limiting factor is a seal ring that is required around CMOS integrated circuits for reliable operation.

The seal-ring is used to avoid die-sawing stress and contaminants into the circuits of the chip as these factors can change the circuit properties and reliability. The circuits of a CMOS chip are thus enclosed by a seal ring structure. In addition, a scribe street surrounds the seal ring structure. This is used for die saw requirements.

The seal ring comprises a contact and via strip, and metal layers surrounding the active area of the chip. The contact and via strip form a continuous ring to block mobile ions from moving into the chip. In addition, p-type diffusion is typically placed underneath the contact as an extra substrate contact.

The seal ring defines the boundary of the customer silicon area. Preferably, there is some spacing between seal ring and the critical active circuitry. Outside the customer area, process control modules (PCMs) are placed by the IC foundry to allow monitoring of process quality.

US 2016/0136686 discloses a hexagonal array of cMUT cells. The hexagonal array results in an offset between adjacent columns. There is an array of transmit-receive cells with the same pitch in both directions as the array of cMUT cells.

US 2005/0094490 discloses various other hexagonal cMUT cell designs again with circuit portions having the same pitch in both directions as the array of cMUT cells.

There remains a problem relating to the seam in the aperture of (large) transducer arrays that can cause image artefacts. Such a seam is needed when a transducer arrangement is to be formed with an aperture size larger than the maximum usable area of a stepper reticle. There also remains a problem in enabling a modular design of the ASICs and cMUT element arrays such that ASICs may be used with different transducer designs.

The invention is directed to some of these issues.

### SUMMARY OF THE INVENTION

The invention is defined by the claims.

According to the invention, there is provided an ultrasound transducer, comprising:
a control circuit which comprises an integrated circuit, the integrated circuit comprising an array of circuit portions;
an array of capacitive micromachined ultrasound transducer, cMUT, elements provided over the top of (or arranged to overlay) the control circuit; and
an array of vias, with at least one via providing a coupling between each cMUT element and a respective one of the circuit portions beneath (the circuit portion said CMUT element is arranged to overlay), those circuit portions together defining a circuit area, wherein the pitch of the array of CMUT elements is greater than the pitch of the circuit portions in at least one direction of the array such that in said at least one direction, the cMUT elements extend beyond one or both sides of the circuit area, wherein the at least one direction of the array comprises a row direction or both row and column directions.

The control circuit is preferably an ASIC. The array of circuit portions is preferably a regular array, i.e. with uniform pitch in the row and column directions.(ignoring any slight inconsequential deviations in pitch which have no intended purpose).

Note that the "circuit area" is intended to mean only the combination of the used circuit portions. Each circuit portion is a unit-cell of the circuit, for performing the same functions, such as delivery of signals to the cMUT element and receipt of signals from the cMUT element. The "circuit area" is thus the combination of the unit cells (which may be considered to be an active part of the physical circuit), which for example has an additional edge around the circuit portions (which may be considered to be a non-active part). This non-active edge may be considered to be another area of the control circuit.

By providing different pitches in the cMUT element array and the array of circuit portions, an overlap can be provided so that the circuits may be separated (etched or diced) while keeping a protective outer region outside the circuit area. When the transducers are tiled, the overlap may be used to ensure that a regular array of cMUT elements is formed, even though the underlying circuit portions form a disrupted array because of the protective outer regions.

There is a preferably a single via between each cMUT element and its respective circuit portion.

The cMUT elements for example each occupy an area and there are spaces defined between the occupied areas, and wherein each via is located at a respective one of the spaces. Thus, the vias may be positioned laterally of the cMUT elements.

Each via for example connects to the circuit portion beneath at a relative position within the shape of the circuit portion, and there are at least two different relative positions such that a first cMUT element connects to its respective circuit portion at a first relative position, and a second cMUT element connects to its respective circuit portion at a second relative position.

Thus, the circuit portions are designed with different formats, to allow connection from the cMUT elements to different positions of the circuit portions. This for example allows each via to be at the same position relative to the cMUT element, or at a limited choice of discrete via positions relative to the cMUT element (because there there is less space for freedom of position selection). The variation in relative position needed by the different array pitches (in one direction or in both orthogonal directions) is thus enabled by making connection to the circuit portions at different relative locations.

The overlap may be all along a side (wherein the "side" of the circuit is intended to mean all four sides, i.e. top edge, bottom edge and both lateral edges), or (in the case of a hexagonal design, discussed below) it may be staggered between opposite sides.

Along each row of cMUT elements, each relative position may be different in the row direction, and the cMUT elements at one end of each row overlap one side of the circuit area in the row direction.

Note that the term "row" is somewhat arbitrary. It is intended simply to indicate one array direction, and for simplicity of language, the orthogonal direction can then simply be referred to as a "column". Each relative position is different because at each position along the row, the difference in pitch creates a different offset.

The cMUT elements at a same end of each row may overlap a same side of the circuit area. Thus, all along a lateral side (at one end of the rows), there is an overlap of the cMUT elements.

Along each column of cMUT elements, each relative position may be different in the column direction, and the cMUT elements at one end of each column overlap one side of the circuit area in the column direction.

In this case, the pitch difference applies in both the row and column directions. Each relative position is different along the column because at each position along the column, the difference in pitch creates a different offset.

The cMUT elements at a same end of each column may overlap a same side of the circuit area. Thus, all along a top or bottom side (at one end of the columns), there is an overlap of the cMUT elements.

In one set of examples, along each row of cMUT elements, the via of a first set of cMUT elements may be located at a space in a first position relative to the cMUT element, and the via of a second set of cMUT elements is located at a second, different, position relative to the cMUT element, and wherein the cMUT elements at each end of each row overlap the respective side of the circuit area in the row direction.

The cMUT elements may have a mirror symmetry between opposite sides. The cMUT elements to the left may use the first relative position and the cMUT elements may use the second relative positon. The cMUT elements in this design overlap both opposite lateral sides of the circuit area.

In another set of examples, the via of each cMUT element may be located at a space at a same position relative to the cMUT element. The cMUT elements in this set of examples can all have the same design. All of the relative position shift caused by the pitch differences is taken up by the position at which the via connects to the circuit portion.

The cMUT element arrays may form a rectangular array. However, the cMUT elements may be staggered between adjacent rows by half a cMUT element pitch. This defines a hexagonal array.

In all examples, the control circuit may have more circuit portions than the number of cMUT elements, such that there are spare circuit portions. These spare circuit portions may for example be located opposite the side where overlap is created, particularly if only two transducers are to be tiled (in that direction). They may assist in simplifying the dicing operation. Thus, the cMUT elements may extend beyond one side of the circuit area and the spare circuit portions are at an opposite side.

The cMUT elements may extend beyond two orthogonal sides of the circuit area and the spare circuit portions are then at both opposite sides. This relates to a version with pitch difference in both orthogonal directions. The spare circuit portions will then be all around a 2x2 array.

The invention also provides an ultrasound transducer arrangement, comprising:
a plurality of ultrasound transducers, at least some of which are as defined above, forming a tiled arrangement,
wherein the control circuits are spaced apart and the cMUT elements of the ultrasound transducers together form a regular array.

Thus, even though the control circuits are spaced apart to simplify dicing of the control circuits, the cMUT elements form a regular array.

The cMUT elements at each end of each row may overlap a same respective overlap row side of the circuit area in the row direction, and the ultrasound transducer arrangement comprises:
a row of two ultrasound transducers as defined above with the overlap row sides facing each other; or
a row of three ultrasound transducers with the outer two as defined above with the row overlap sides facing each other, and a further ultrasound transducer in the middle.

Thus, a row of two or three ultrasound transducers may be formed.

The ultrasound transducer arrangement may instead comprise a 2x2 array of ultrasound transducers each as defined above. Thus, a 2D array of ultrasound transducers may be formed.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known design of cMUT element (a so-called cell) for use in an ultrasound system and a known drive arrangement;
Figure 2 shows the cMUT element with a first degree of membrane collapse;
Figure 3 shows the cMUT element with a second first degree of membrane collapse;
Figure 4 shows a first possible arrangement to connect to an underlying ASIC;
Figure 5 shows a second possible arrangement to connect to an underlying ASIC;
Figure 6 shows a control circuit for a 4 x 6 transducer element matrix array;
Figure 7 shows the same circuit of Figure 6 after processing of the cMUT elements;

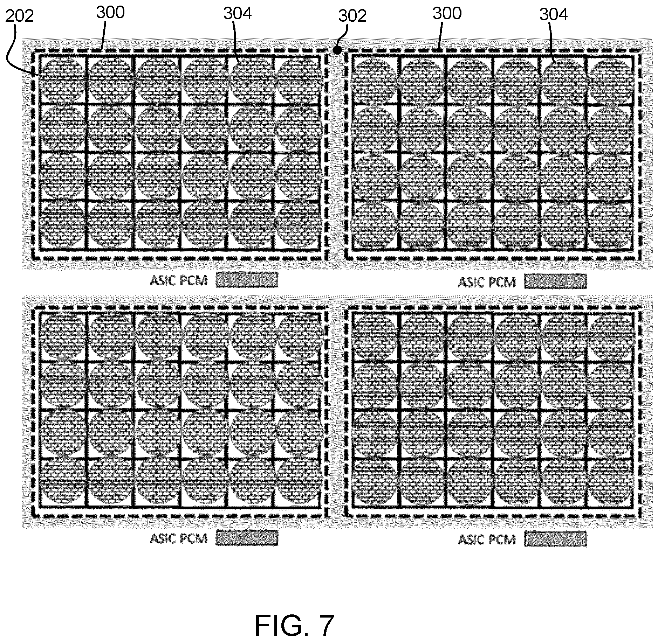

Figure 7 shows the conventional layout with identical cMUT pitch and pitch of the control circuit portions;

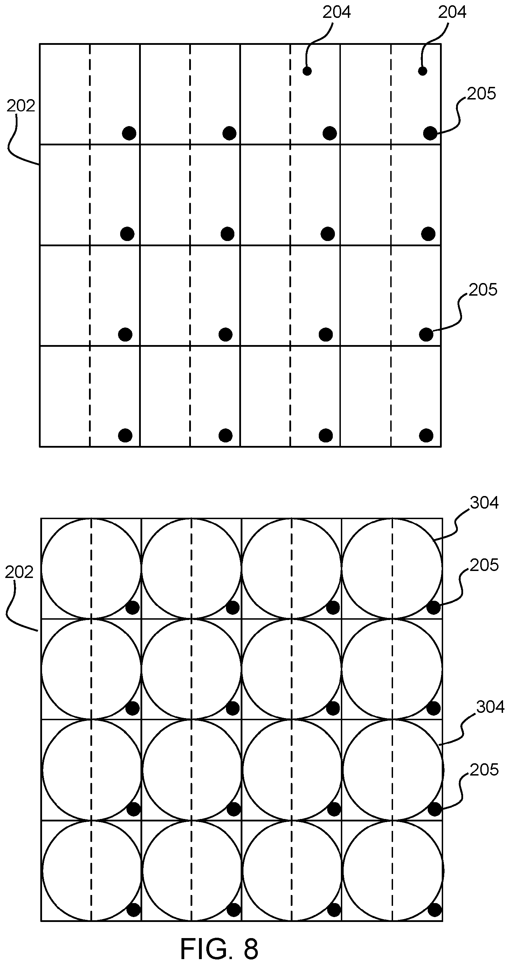

Figure 8 shows the conventional ASIC design having a regular array of circuit portions as wells as the cMUT positioning over the top;

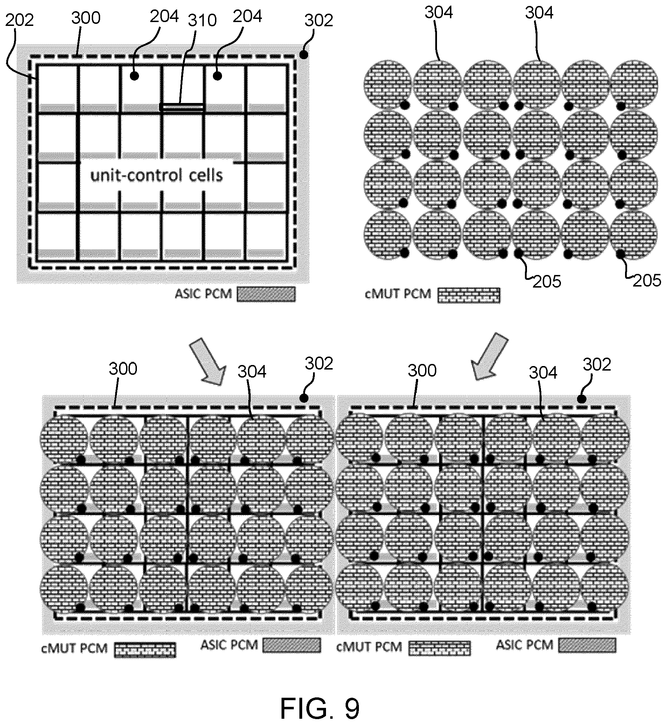

Figure 9 shows a first example of circuit portions having a smaller area and hence smaller pitch than the area of the cMUT elements;

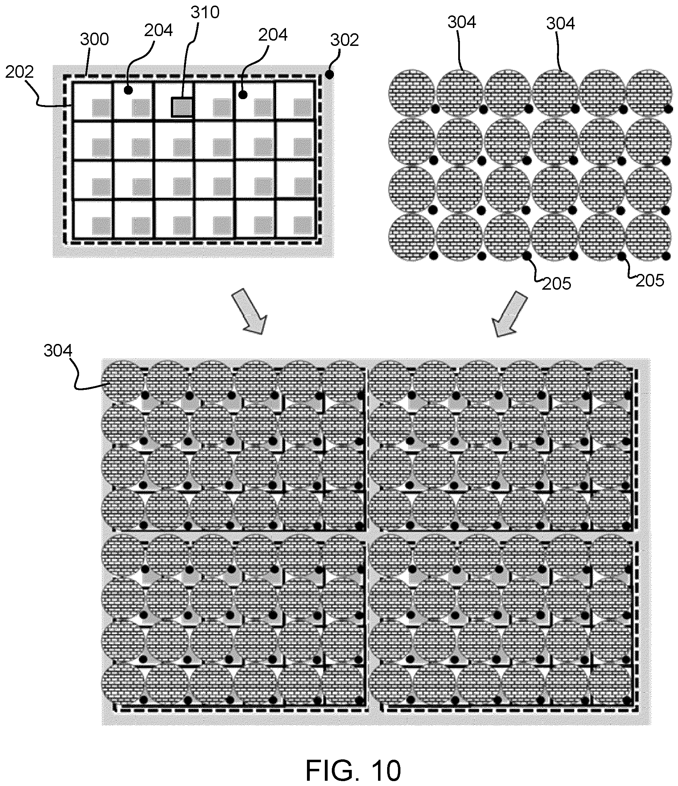

Figure 10 shows a second example;

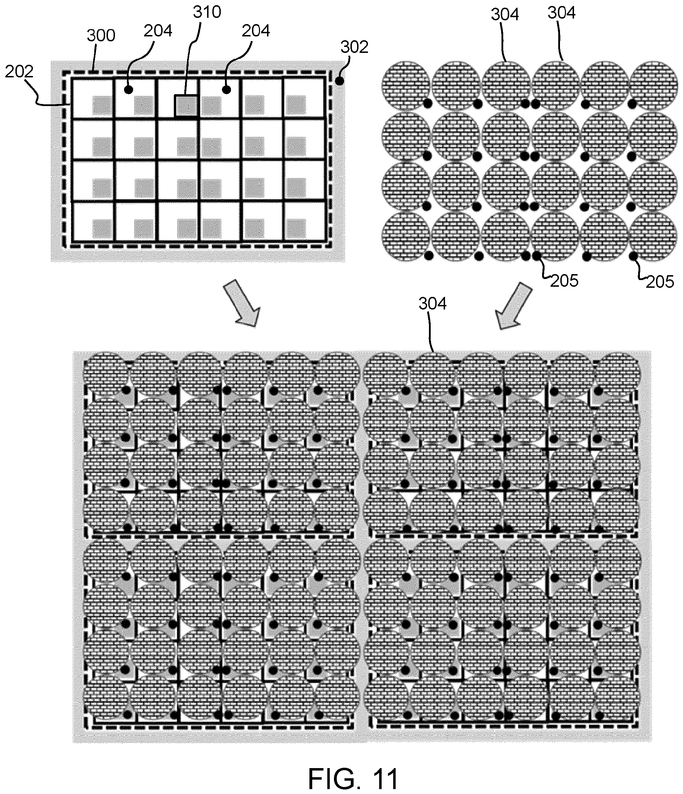

Figure 11 shows a variant of the design of Figure 10;

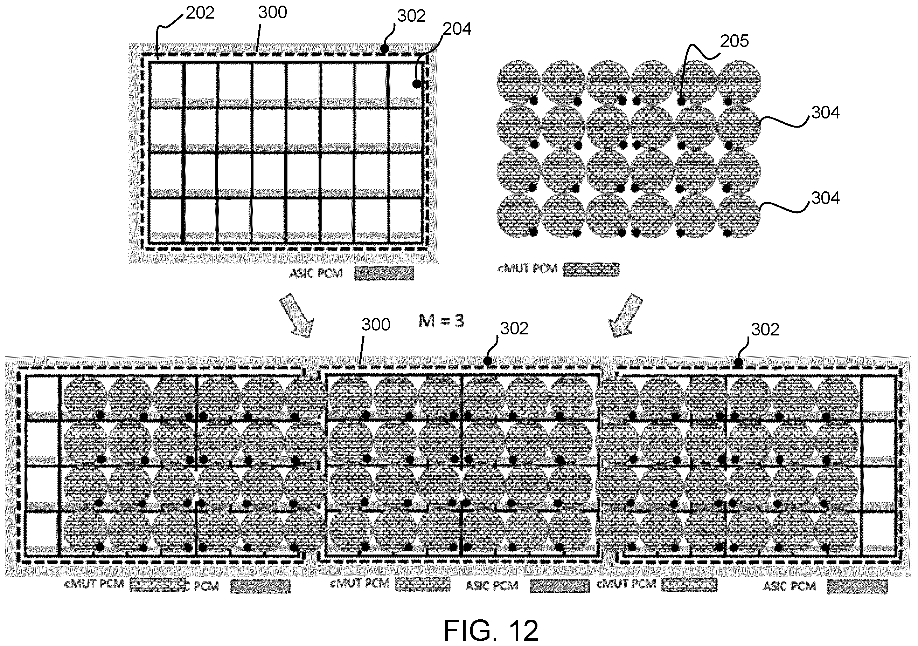

Figure 12 shows an example in which the control circuit has more circuit portions than the number of cMUT elements, such that there are spare circuit portions;

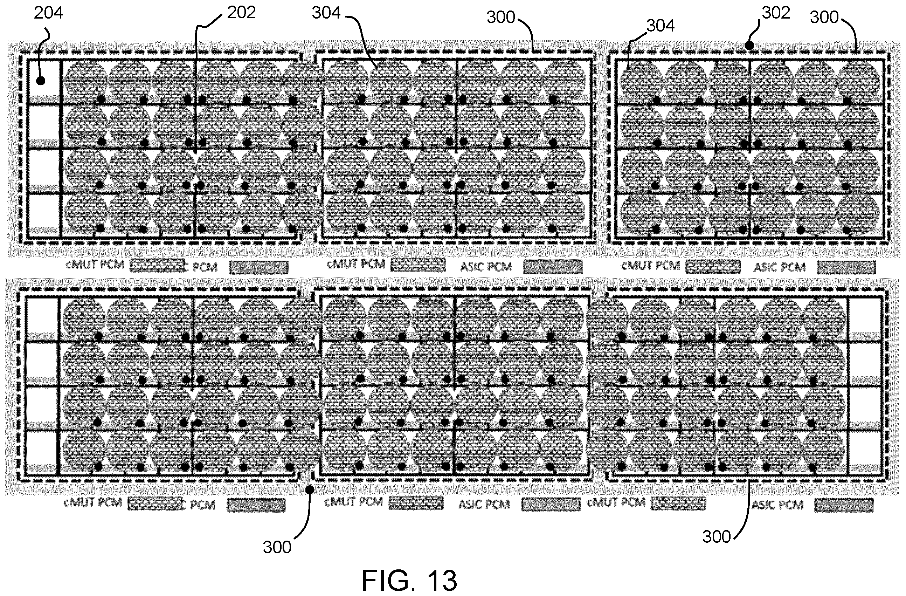

Figure 13 shows that the design of Figure 12 enables transducer arrays with different sizes on one wafer;

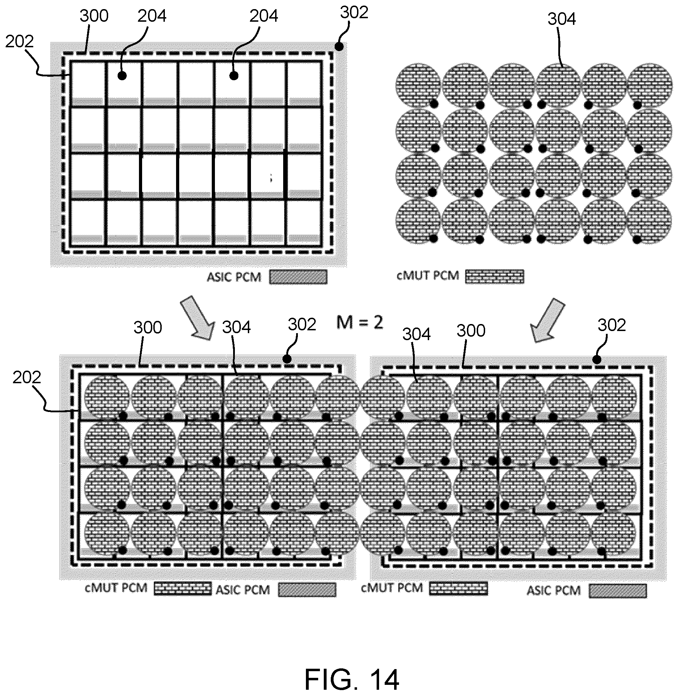

Figure 14 shows another implementation with spare circuit portions;

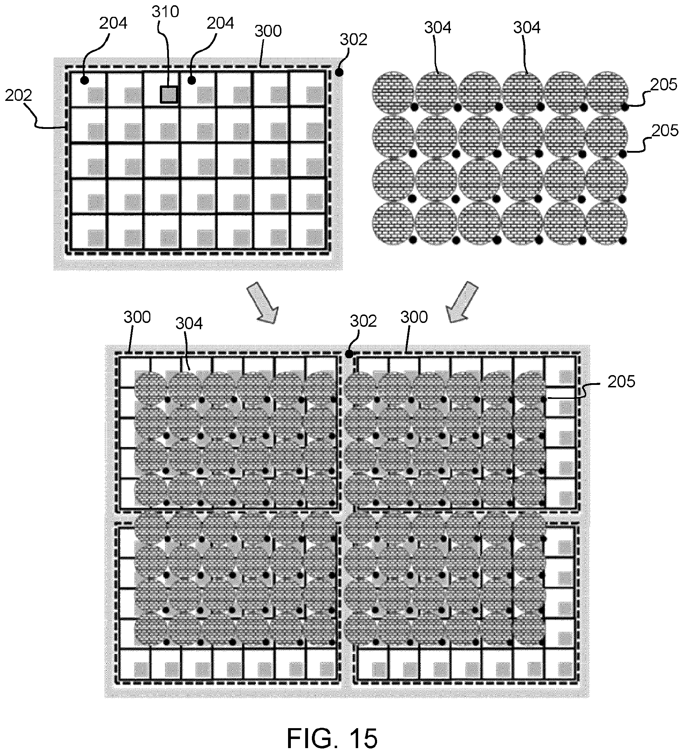

Figure 15 shows another example with spare circuit portions;

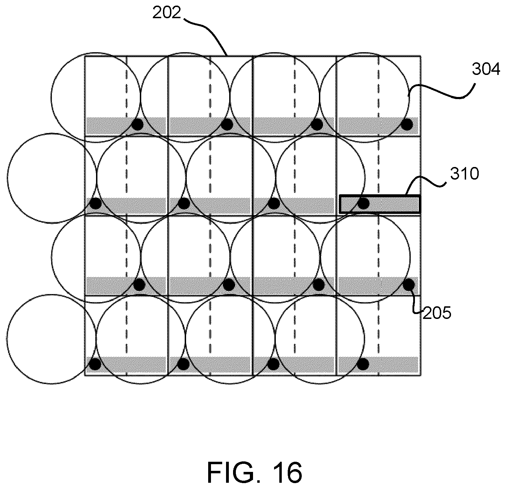

Figure 16 shows rows of cMUT element staggered by half a cMUT element pitch, to form a hexagonal array of cMUT elements;

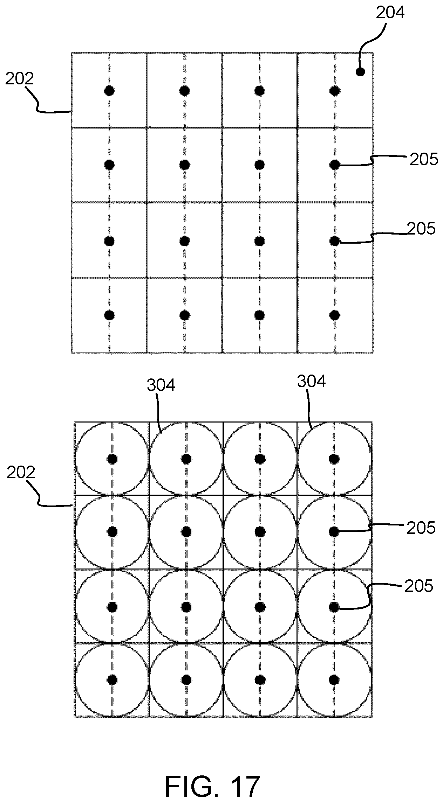

Figure 17 shows a control circuit with contact pads for centrally located vias and the cMUT array over the top;

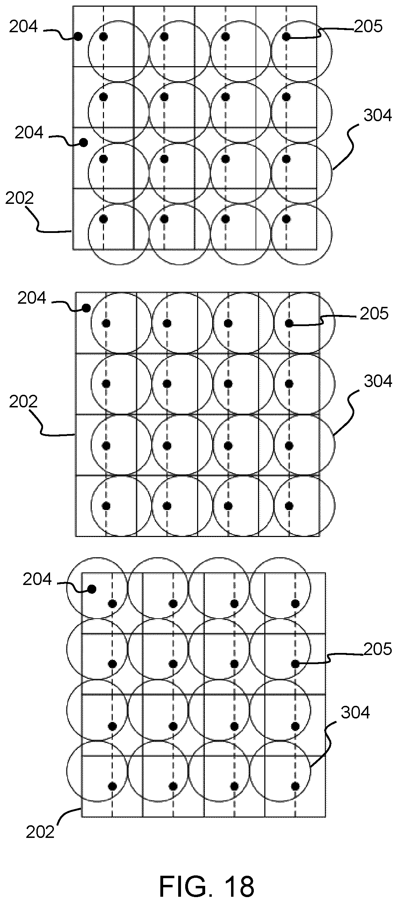

Figure 18 shows three possible configurations with cMUT elements shifted relative to the circuit portions;

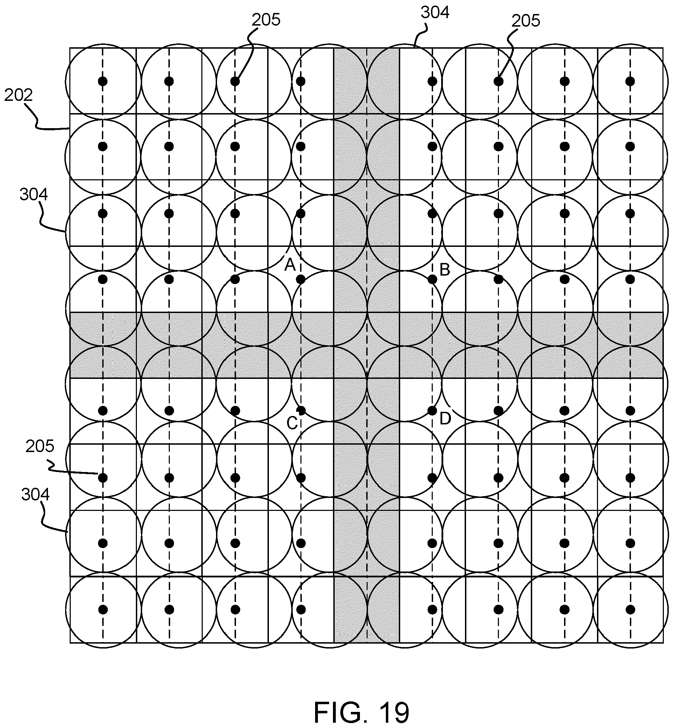

Figure 19 shows an example in which the transducer elements are chosen to be larger than the underlying control circuit portions;

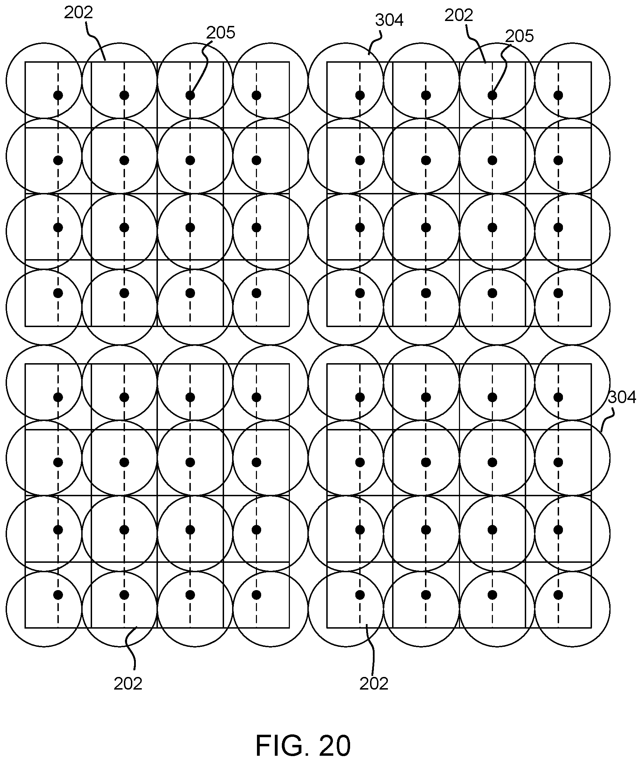

Figure 20 shows four separate modules butted together as a 2x2 array or a continuous transducer array over multiple adjacent control circuits;

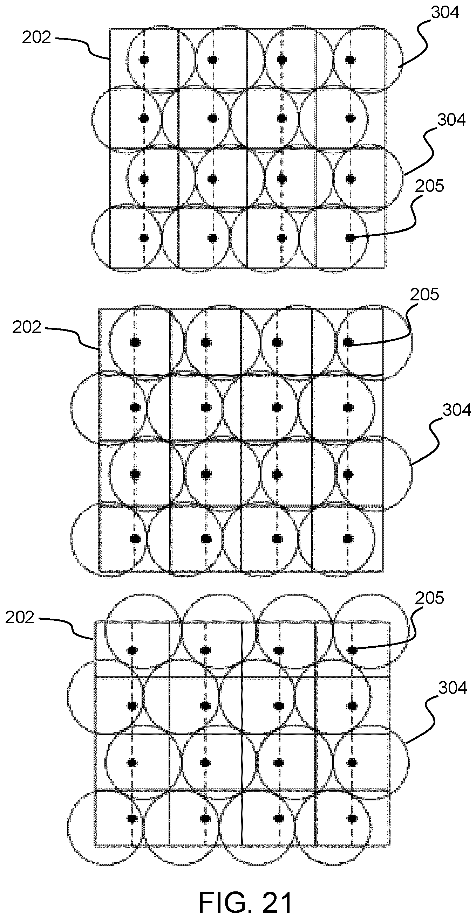

Figure 21 shows three examples in which the cMUT elements of one row are staggered by half a pitch compared to the adjacent rows; and

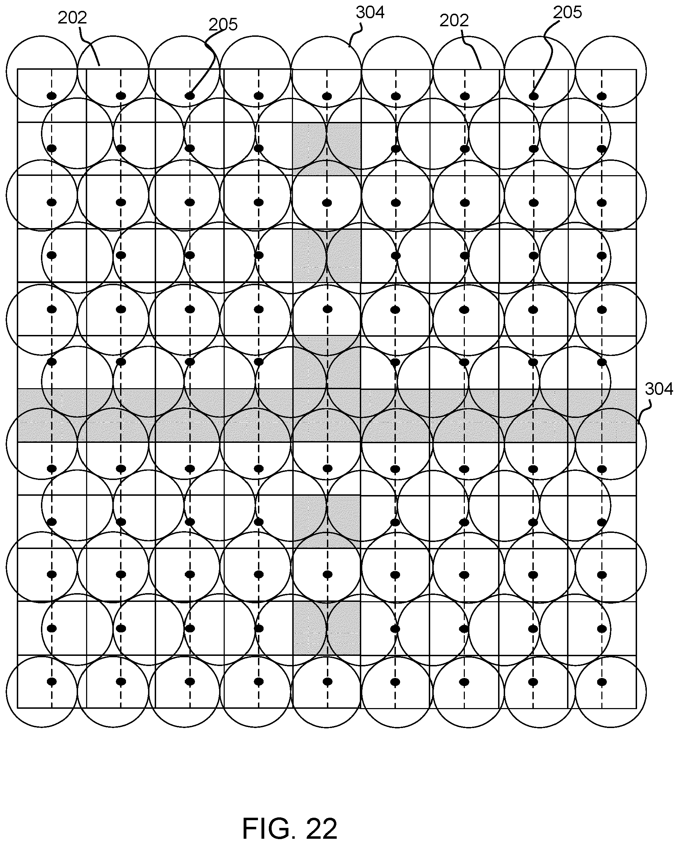

Figure 22 shows an example of a larger hexagonal transducer array of the type shown in the bottom of Figure 21.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound transducer having a control circuit with a regular array of circuit portions which together define a circuit area. cMUT elements are provided over the top of the control circuit. Vias provide a coupling between each cMUT element and a respective one of the circuit portions beneath (the circuit portion, which is being overlaid by the CMUT element). The vias are located at spaces between the cMUT elements and are connected to the circuit portion beneath at a position relative to the shape of the circuit portion. It shall be understood in the context of the present application that term beneath is defined as a projection of a surface area of one element onto another unit, which said element is arranged to overlay. Thus, beneath bottom electrode shall be interpreted as within the projection of the electrode's surface area onto the surface area of the circuit portion the CMUT element is arranged to overlay. The pitch of the array is greater than the pitch of the circuit portions in at least one direction of the array such that in said at least one direction, the cMUT elements extend beyond one or both sides of the circuit area. This makes tiling easier and enables a modular design.

The invention makes use of cMUT transducer elements. cMUT transducer elements can be made by semiconductor processes and in particular the same processes used to produce the application specific integrated circuits (ASICs) needed by an ultrasound probe such as a CMOS process. The manufacture of cMUT transducer elements thus does influence the ASIC parameters.

cMUT elements are tiny diaphragm-like devices with electrodes that convert the sound vibration of a received ultrasound signal into a modulated capacitance. Many of such individual cMUT elements can be connected together and operated in unison as a single transducer element. For example, four to sixteen cMUT elements can be coupled together to function in unison as a single transducer element.

The manufacture of cMUT-based ultrasound systems is therefore more cost-effective compared to PZT-based systems. Moreover, due to the materials used in such semiconductor processes, the cMUT elements exhibit much improved acoustic impedance matching to water and biological tissue, which obviates the need for (multiple) matching layers and yields an improved effective bandwidth.

cMUT elements in particular are able to function over a broad bandwidth, enable high resolution and high sensitivity imaging, and produce a large pressure output so that a large depth of field of acoustic signals can be received at ultrasonic frequencies.

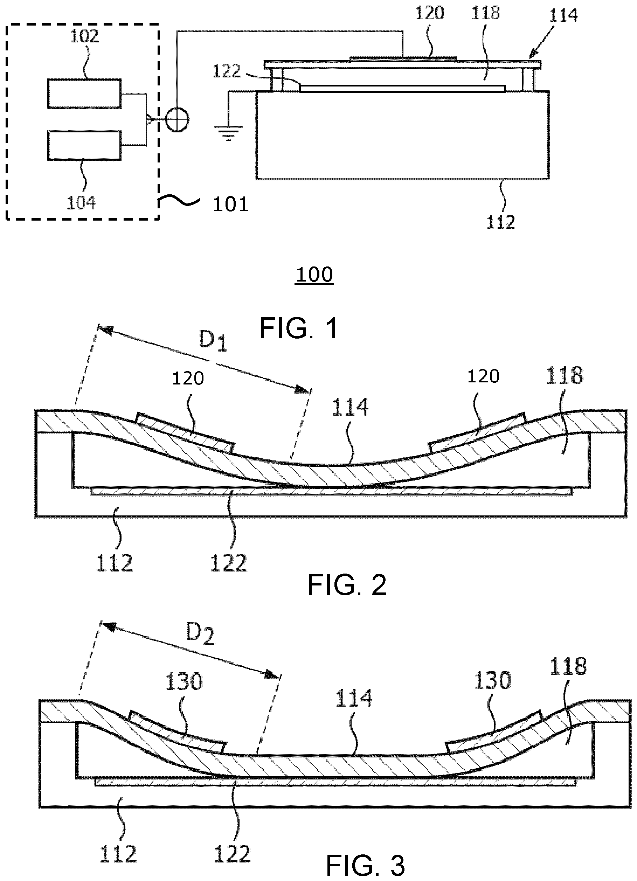

Figure 1 shows a known design of cMUT element 100 (a so-called cell) for use in an ultrasound system and a known drive arrangement 101. The cMUT element 100 comprises a flexible membrane or diaphragm 114 suspended above a silicon substrate 112 with a gap or cavity 118 there between. A first electrode 122 is located on the floor of the cell on the upper surface of the substrate 112 in this example. A second electrode 120 is located on the diaphragm 114 and moves with the diaphragm. In the example shown, the two electrodes are circular.

A dielectric (not shown) is provided on the substrate 112 and underneath the top (second) electrode 120. The diaphragm may instead function as a dielectric layer.

Preferably, there are two dielectrics which may be equal in composition and thickness, but may be also asymmetric (different materials and thicknesses).

The membrane layer 114 is fixed relative to the top face of the substrate layer 112 and configured and dimensioned so as to define a spherical or cylindrical cavity 118 between the membrane layer 114 and the substrate layer 112.

Other realizations of the electrode 120 design can be considered, such as electrode 120 may be embedded in the membrane 114 or it may be deposited on the membrane 114 as an additional layer.

The first electrode may be directly exposed to the gap 118 or separated from the gap 118 by an electrically insulating layer or film to prevent a short-circuit between the second electrode 120 and the first electrode 122.

In Figure 1 the first electrode 122 is grounded by way of example. Other arrangements, e.g. a grounded second electrode 120 or both second electrode 120 and first electrode 122 floating are of course equally feasible.

The electrodes of the cMUT element 100 provide the capacitive plates of the device and the gap 118 is the main dielectric between the plates of the capacitor. When the diaphragm vibrates, the changing dimension of the dielectric gap between the plates provides a changing capacitance which is sensed as the response of the cMUT element 100 to a received acoustic echo.

The spacing between the electrodes is controlled by applying a static voltage, e.g. a DC bias voltage, to the electrodes with a voltage supply 101. The voltage supply 101 may optionally comprise separate stages 102, 104 for providing the DC and AC or stimulus components respectively of the drive voltage of the cMUT elements 100, e.g. in transmission mode. The first stage 102 may be adapted to generate the static (DC) voltage component and the second stage 104 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the cMUT element 100 into its collapsed state. This applies for a collapsed mode of operation. The cMUT element may however equally be operated in a non-collapsed mode.

The first stage 102 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

It is known that by applying a static voltage above a certain threshold, the cMUT element 100 is forced into a collapsed state in which the membrane 114 collapses onto the substrate 112. This threshold value may depend on the exact design of the cMUT element 100 and is defined as the DC bias voltage, known as the collapse voltage, at which the membrane 114 sticks to (contacts) the cell floor through the force due to the electric field between the electrodes. The amount (area) of contact between the membrane 114 and the substrate 112 is dependent on the applied bias voltage.

Increasing the contact area between the membrane 114 and the substrate 112 increases the resonant frequency of the membrane 114, as will be explained in more detail with the aid of Figure 2 and Figure 3.

The frequency response of a collapsed mode cMUT element 100 may be varied by adjusting the DC bias voltage applied to the cMUT electrodes after collapse. As a result, the resonant frequency of the cMUT element increases as a higher DC bias voltage is applied to the electrodes.

The principles behind this phenomenon are illustrated in Figures 2 and 3. The cross-sectional views of Figures 2 and 3 illustrate this one-dimensionally by the distances D1 and D2 between the outer support of the membrane 114 and the point where the membrane begins to touch the floor of the cavity 118 in each illustration. It can be seen that the distance D1 is a relatively long distance in Figure 2 when a relatively low bias voltage is applied, whereas the distance D2 in Figure 3 is a much shorter distance due to a higher bias voltage being applied. These distances can be compared to long and short strings which are held by the ends and then plucked. The long, relaxed string will vibrate at a much lower frequency when plucked than will the shorter, tighter string. Analogously, the resonant frequency of the cMUT element in Figure 2 will be lower than the resonant frequency of the cMUT element in Figure 3 which is subject to the higher bias voltage.

Thus, a typical cMUT design comprises a flexible membrane or diaphragm suspended above a silicon substrate with a gap or cavity between them. The gap is caused by removing a sacrificial layer during manufacture. A first electrode is located on the floor of the cell on the upper surface of the substrate and a second electrode is located on the diaphragm and moves with the diaphragm.

The operation of cMUT elements will be well known to those skilled in the art.

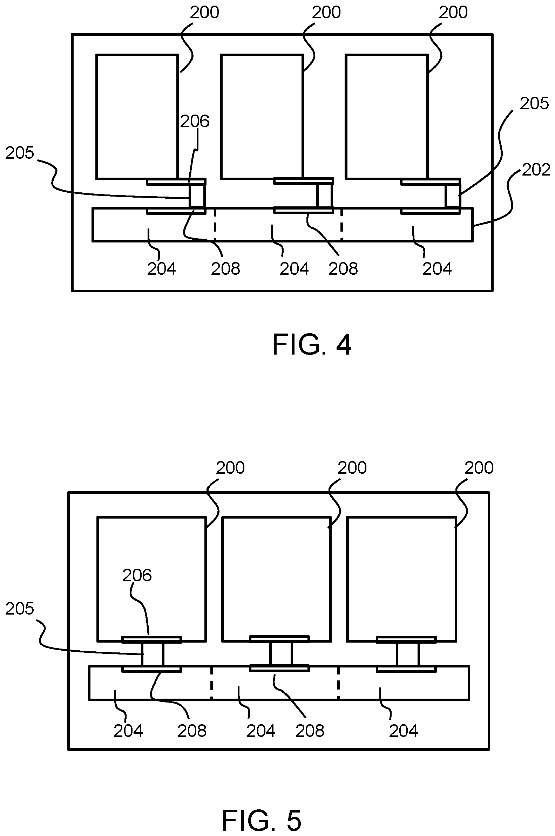

To connect to an underlying ASIC, vias are used. A first possible arrangement is shown schematically in Figure 4.

The cMUT transducer elements 200 are provided over the control circuit 202 (an ASIC integrated circuit), the integrated circuit comprising a regular array of circuit portions 204 (these are the unit cells of the circuit). There is a circuit portion associated with each cMUT element. The cMUT elements are provided over the top of the control circuit 202.

The cMUT elements each occupy an area depending on the shape of the cMUT elements. Typically, they are circular, so that there are spaces defined between the occupied circular areas.

Vias 205 are provided in these spaces, for connecting between signal electrode pads 206 of the cMUT cell and contact pads 208 of the circuit portions. Only one via is needed, for example the via from the control circuit portion connects to the bottom electrode of the cMUT, and the cMUT top electrodes are all connected in parallel to a common ground or bias potential. The vias are located laterally to minimize the surface topography below the cMUT membranes, i.e. to keep the surface below the cMUT membranes as flat as possible.

A second possible arrangement is shown schematically in Figure 5. The cMUT transducer elements 200 are again provided over the control circuit 202 with its regular array of circuit portions 204.

Vias 205 are provided beneath the cMUT elements in this design, again for connecting between a signal electrode pad 206 of the cMUT cell and a contact pad 208 of the circuit portions.

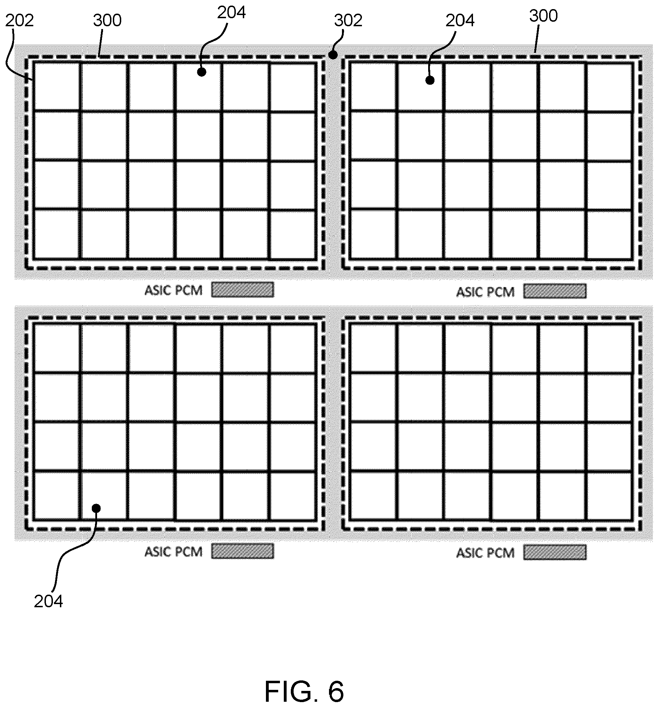

Figure 6 shows a set of four control circuits 202 each for a 4 x 6 transducer element matrix array. The small array dimensions are chosen for simplicity only. The ASIC comprises a regular array of circuit portions 204/

From an ASIC design perspective, it is difficult to create 2D arrays of unit-cells (i.e. circuit portions) that do not consist of perpendicular rows and columns, as this severely complicates interconnect routing. Therefore, a 2D array of ASIC unit-control cells having perpendicular rows and columns is assumed. The height and width of each ASIC unit cell are not necessarily equal.

A seal ring 300 surrounds the critical circuit components and a saw street 302 for die separation is around the outside of the seal ring 300. Figure 6 also shows the ASIC process control modules (PCMs).

Figure 7 shows the same circuit of Figure 6 after processing of the cMUT elements 304 (in a MEMS cleanroom). Each cMUT element 304 is indicated by a circle, which is the prevalent shape of cMUT transducer elements, although the invention is not limited to circular transducer elements only. In all examples, the bottom electrode is assumed to have equal outer dimensions as the transducer element for ease of discussion.

Figure 7 shows the conventional layout with identical cMUT pitch and pitch of the control circuit portions.

A first set of examples is based on the use of vias which are placed next to the transducer elements as explained with reference to Figure 4. This is standard practice for current cMUT transducers.

The top part of Figure 8 shows the conventional ASIC design having a regular array of circuit portions, each receiving electrical connections from a via 205 (so there is an associated contact pad at the ASIC). The vias are all at the same position relative to the control circuit portions, i.e. in the bottom right corner in this example.

The bottom part of Figure 8 shows the cMUT elements 304 over the top, and thus corresponds to Figure 7 but also shows more clearly the via positioning in the spaces not occupied by the cMUT elements.

A first aspect is based on the use of a control circuit (ASIC) with circuit portions having a smaller area and hence smaller pitch than the area of the cMUT elements. The size difference is such that the seal ring 300 fits together with the active parts of the control circuit (i.e. the array of circuit portions) under the transducer element array.

Figure 9 shows a first example.

In this example, the pitch of the cMUT element array is greater than the pitch of the circuit portions only in a row direction. Thus, in this row direction the cMUT elements extend beyond the circuit area of the control circuit defined by the combination of the unit cells of the control circuit. In this example the cMUT elements extend beyond the circuit area at both sides (i.e. at both ends of the rows).

The top left image shows the control circuit as a regular array of identically sized control circuit portions 204 and the seal ring 300 and saw street 302.

The top right image shows the array of cMUT elements 304. It also shows the cMUT PCM.

The bottom image shows that the overlap enables the individual full control circuits 202 (i.e. one full array of the control circuit portions) and associated cMUT elements to be tiled while maintaining a uniform array of cMUT elements. This example shows two identical transducers arranged side by side to form the transducer arrangement. The two sides which are butted together each have the overlap of the cMUT elements.

A row of any number of transducers may be formed, since the overlap is at both ends of the rows.

The result of the difference in pitch is that different cMUT elements connect to their respective circuit portions at different relative positions. The top left image shows a connection region 310 of each circuit portion. Different vias 205 connect to different positions within that general connection region.

The circuit portions 204 are identical in size to cMUT elements in one dimension (the column direction, for example corresponding to the elevation scanning dimension) and are slightly smaller in the other dimension (the row direction, for example corresponding to the azimuthal scanning dimension). For example, in case of a 32 x 32 cMUT element array, the area of the transducer element could be 300 x 300 µm, while the area of the circuit portion 204 could be 300 x 285 µm.

This causes the overall transducer to exceed the ASIC control area by +480µm in the azimuthal direction while there is no excess in elevation direction. If the center of the transducer module is aligned with the center of the control circuit, the transducer module will overlap the control circuit by +240 µm in both opposite row directions. This is sufficient to close the gap between the active areas of two control circuit dies (i.e., the margin to the seal ring, the seal ring width and the saw street width).

In this example, the transducer element array is split in two half arrays; the left half and the right half. The vias for the left half are located on the lower right corner of the cMUT element area and the vias for the right half are located on the lower left side of the cMUT element area.

Thus, the via of a first set of cMUT elements is located at a space in a first position relative to the cMUT element (lower left), and the via of a second set of cMUT elements is located at a second, different, position relative to the cMUT element (lower right). As a result, there is a double column of vias in the middle of the module. The alignment of the cMUT element array and the control circuit is such that the double vias connect to different circuit portions.

The via processing can be part of the IC or the MEMS processing stages. As the via pitch primarily is linked to the transducer element pitch, it is preferred to realize the via during the MEMS processing. The control circuit portions allow "landing" of the via on every X-coordinate position in the circuit portion, i.e. anywhere within the connection region 310. The connection regions for example comprises a horizontal metal track extending from one side of the cell to the other side to allow such connection.

In this way, the circuit portions can have identical designs even though connections to the circuit portions can be made at different positions.

Figure 10 shows a second example. In this example, the vias are all located at a same position relative to the cMUT element (lower right in this example). In this example, no special arrangement of vias is needed at the cMUT element level.

In Figure 10, the cMUT elements overlap only at one of a pair of opposite sides. However, Figure 10 shows that the pitch of the circuit portions is also less than the pitch of the cMUT elements in the column direction. The position of the via relative to the control circuit portions is thus also different in the column direction, so that the cMUT elements at one end of each column overlap the corresponding (top or bottom) one side of the circuit area in the column direction.

According to the invention, the pitch difference applies in both the row and column directions.

The connection region 310 becomes two dimensional because the position at which connection is made varies in both the row direction and the column direction.

In Figure 10, the cMUT elements at a same end of each column overlap a same side of the circuit area. Thus, all along a top or bottom side, there is an overlap of the cMUT elements.

As an example, in the case of a 32 x 32 element array, the area of the transducer element could be 300 x 300 µm, while the area of the circuit portion 204 could be 295 x 295 µm.

If the right lower corner of the transducer element is aligned with the right lower corner of the control circuit as shown, the transducer module will overlap the control circuit by 160 µm on the left and the upper sides.

The control circuit portions must allow "landing" of the via in the connection region 310. For this, the circuit portions contain a relatively large metal plate area to allow for such connection. The shape of the metal area can be square but also oval/rectangular/triangular or indeed any other shape.

Figure 11 shows a variant of the design of Figure 10 that uses the via arrangement explained with reference to Figure 9 in combination with the two-axis pitch adjustment of Figure 10.

The advantage of this combination of features version is that smaller "landing metal" is required in the connection regions 310 or larger transducer overlaps can be realized.

To harvest the transducer modules from the wafer, a form of wafer level die separation such as dicing or etching is required. Due to the modular approach, it is possible to harvest transducer arrays with various sizes from one wafer.

Harvesting the transducer arrays can be done using mechanical or chemical dicing (etching). When etching, narrow dicing/etch streets can be used (e.g. < 10µm). It is for example possible to design the transducer module such that the street approximately fits the normal kerf between the transducer elements.

In the case of mechanical dicing (sawing), large streets are needed. In this case, it is possible to sacrifice transducer modules in order to accommodate the required dicing street; this route may be a viable option if the transducer array consists of many transducer modules (if small modules are used).

Often, transducer arrays use dummy elements on the rim of the arrays. The dummy components may not however be needed. If dicing impacts the characteristics of the rim transducer elements, the control circuit should be aware of this and not activate the outer elements in the application.

The examples above all have the same number of circuit portions as cMUT elements.

Figure 12 shows an example in which the control circuit has more circuit portions than the number of cMUT elements, such that there are spare circuit portions.

The control circuit again has circuit portions 204 having an area slightly smaller than the area of the transducer elements (in one direction only for the example of Figure 12 but the size difference can be in both directions).

The top left image shows an 8x4 array of circuit portions whereas the cMUT array at the top right has 6x4 elements. The cMUT array has the design of Figure 9 with two possible via positions relative to the cMUT element.

The area of overlap can correspond to the width of a circuit portion. Thus, in the transducer arrangement of three transducers shown at the bottom, the left transducer has an overlap to the right and there is a full column of spare control circuit portions, the right transducer has an overlap to the left with a column of spare circuit portions to the right and there is a different design of transducer in the middle with no overlap. The overlaps from each side are sufficient to cover both adjacent seal rings.

The arrangement shown at the bottom of Figure 12 is single die and it is not to be diced because it is fully occupied with transducer elements.

For example, a 32 x 32 element array may be connected to an control circuit 202 with (31+N) x (31+M) circuit portions 204 (in the elevation column and azimuthal row directions respectively). M and N are integer numbers; their value determines the maximum number of transducer modules that can be combined in a transducer array that does not extend the seal rings on the rim.

For example, with N = 2 and M=2 (1 extra row and 1 extra column of circuit portions), transducer arrays composed of 1 or 2 modules in elevation and 1 or 2 modules in azimuthal direction can be realized without extending the seal rings on the outer side of the array.

The example of Figure 12 shows a 3-module array with M=3 and N=1 (2 extra columns of circuit portions per control circuit).

By using a special stepper job, the alignment of the transducer array and the control circuit portions can be changed. In the example of Figure 12, the center of the middle transducer is aligned with the center of the corresponding ASIC control circuit, the center of the left transducer is shifted to the right and the center of the right transducer module is shifted to the left relative to the center of the corresponding ASIC control circuit.

Thus, the control circuits may still all have the same design and the cMUT element arrays may still have the same design.

There are spare circuit portions in this arrangement, so that not all circuit portions are needed by the transducer elements. The system controller will be aware of this and not stimulate these control units.

Figure 13 shows that this modular design enables transducer arrays with different sizes on one wafer.

In Figure 13, each control circuit has 8 columns of circuit portions and the cMUT array has 6 columns (M=3, 2 extra columns of circuit portions).

Figure 13 shows in the top left a 2 transducer arrangement and in the top right a single transducer arrangement, formed on a single wafer. The wafer may then be diced to separate the two transducer arrangements. In particular, a dedicated dicing street is generated between the two transducer arrangements, as shown. This dicing street simplifies the dicing of the transducer arrays, which can be done mechanically and chemically without real yield loss.

Figure 13 shows in the bottom a 3 transducer arrangement (corresponding to Figure 12) formed on the same wafer design.

Figure 14 shows an example implementation with M=2, N=1 (1 extra column of circuit portions). In case M = even, there is no "middle" transducer module and none of the transducer modules is exactly aligned with the ASIC modules.

In this example, the center of the left transducer is shifted to the right and the center of the right transducer module is shifted to the left relative to the center of the associated ASIC control circuit.

The two transducers are formed on a single wafer.

Figure 15 shows an example with M=2 and N=2 (1 extra column of circuit portions and 1 extra row of circuit portions). In this example, the cMUT element array of the type shown in Figure 10 is used, with a uniform via arrangement relative to the cMUT elements.

A 2x2 array of transducers is thereby formed.

It can be seen that one use of the overlap and pitch arrangement is to enable multiple diced transducers to be tiled, e.g. Figures 9 to 11. Another use is to allow a modular design of control circuit on a single wafer, e.g. Figures 12 to 15. The designs of Figures 12 to 15 are not tiled separate dies, they are formed on a single wafer. However, the pitch mismatch enables different pitches of cMUT element arrays to be formed over the control circuit.

The examples above make use of the mismatch between the size/pitch of the control circuit portions and cMUT elements to enable overlap along one or more sides of a transducer, so that transducers can be tiled or so that a modular design may be employed.

The approach may also be used to enable hexagonal transducer arrays to be formed on top of rectangular arrays of control circuit portions. The well-known advantage of hexagonal transducer arrays over square packed arrays is their higher density of active transducer area. This allows to generate higher pressures, higher receive sensitivity [A/pa] and larger bandwidth.

Figure 16 shows rows of cMUT element staggered by half a cMUT element pitch, to form a hexagonal array of cMUT elements. The via 205 is always at the bottom right. The pitch of the cMUT elements is larger in only the row direction than the circuit portions. This requires a long horizontal connection region 310.

The examples above create flexibility in placing the transducer elements at different positions relative to the underlying control circuit portions. Some designs require special via arrangements, but there is a downside that this complicates the ASIC design, and requires changes to the actual via locations, which differ from one transducer design to another.

Another set of examples uses a further or alternative method to create flexibility in placing the transducer elements at different positions relative to the underlying ASIC unit circuit portions. This set of examples is based on the approach shown in Figure 5, according to which each via to control circuit portion is placed directly below the bottom electrode of the transducer element. This is feasible if the substrate including the via is sufficiently flat for building the transducer element directly on top of it.

In practice, a surface topography well below 50 nm is required at the location of the transducer elements. The relatively large dimension of the transducer element's bottom electrode compared to the via dimension allows shifting of the transducer element relative to the via. Thus, the position of the via can then be kept fixed relative to that of the corresponding ASIC unit control-cell. Therefore, it creates flexibility in placing the transducer elements at different positions relative to the underlying control circuit portions.

This approach may be combined with the approaches described above in order to achieve even greater position flexibility of the transducer elements relative to the underlying control circuit portions.

This set of examples make use of flexibility in placing the cMUT elements at different positions relative to the underlying circuit portions of the control circuit. The vias are directly below the bottom electrode of the transducer element, e.g. located centrally. By shifting the transducer element relative to the via, so that the via is not central to the cMUT element, the cMUT element is consequently shifted relative to its control circuit portion. By intentionally changing the transducer element locations of adjacent ASIC unit control cells, the same ASIC design can be used to build transducers having different center frequencies and/or different apertures.

Figure 17 shows at the top a control circuit formed of control circuit portions 204, with contact pads for centrally located vias 205. This is for example purposes only. The bottom image shows the cMUT elements 304 mounted in conventional manner, with the same array pitch as the control circuit portions, and symmetrically positioned so that the area of each cMUT element overlaps and corresponds with the area of the circuit portion beneath.

Figure 18 shows three possible configurations in accordance with this set of examples. The cMUT elements 304 have the same pitch as the circuit portions 204 but they are offset from alignment so that the cMUT elements overlap one side of the circuit area in the row direction (middle figure) or overlap one side in the row direction and one side in the column direction (top and bottom figures). The connection of the via to the cMUT element is at a different position relative to the cMUT element in these three examples. As the bottom electrodes are assumed to have equal outer dimensions to the transducer elements, it can be seen that in all cases the bottom electrodes overlap with the vias to the corresponding control circuit portions, and therefore they are considered to make electrical contact.

Figure 19 shows an example in which the transducer elements are chosen to be larger than the underlying control circuit portions, in the same way as explained above. This example shows the pitch of the cMUT elements being greater in both orthogonal array directions. This results in a two-way pitch mismatch which can be solved by translating the transducer elements relative to their corresponding control circuit portions, and by skipping over individual control circuit portions in a regular pattern.

The skipped circuit portions are shown in Figure 19 in grey. These circuit portions are not used by the cMUT elements. They form a central row and a central column.

As a result, the circuit area (i.e. the combination of circuit portions associated with the cMUT elements) becomes the four square corners. The cMUT elements overlap these four areas over the skipped circuit portions.

In this way, the design of the cMUT array may be adapted while maintaining the same circuit design. This approach can be extended over larger areas than shown in Figure 19 by simple repetition.

In this particular case, the vias A, B, C and D are just outside the area of the transducer element so that not all vias are directly beneath the cMUT lower electrode. This can easily be overcome by including a trace between the via and the bottom membrane of the corresponding transducer element.

This approach also enables tiling to form a continuous transducer array created from multiple control circuits and cMUT arrays.

Figure 20 shows four separately diced modules butted together as a 2x2 array. In this case, the cMUT elements overlap all four sides of the underlying circuit area, and hence overlap the seal ring. A uniform cMUT element array is formed even though there are gaps between the control circuits 202, in the same way as explained above.

Figure 20 may also be considered to show a transducer formed over multiple ASIC modules on a single wafer.

As for the examples above, this approach also allows hexagonal transducer arrays on top of rectangular arrays of control circuit portions.

Figure 21 shows three examples in which the cMUT elements of one row are staggered by half a pitch compared to the adjacent rows.

The top image shows an example in which the cMUT elements have the same pitch in both orthogonal directions as the circuit portions. Odd rows are shifted slightly to the right and even rows are shifted slightly to the left.

The middle image shows a difference in pitch only in the row direction, so the cMUT element pitch is greater in the row direction than the circuit portions.

The bottom image shows a difference in pitch in the row and column directions, so the cMUT element pitch is greater in the row and column directions than the circuit portions. Transducer element arrangements other than hexagonal or square transducer are also possible

The staggered cMUT configurations may need additional ASIC programming in order to generate the correct (delay) control of transducer elements in the "odd" columns relative to the "even" columns.

Figure 22 shows an example of a larger hexagonal transducer array of the type shown in the bottom of Figure 21 (with larger pitch in both directions) on a rectangular ASIC control circuit. The skipped control circuit portions are again depicted in grey. They are positioned along (but not fully occupying) a central row and a central column.

As a result, the circuit area (i.e. the combination of circuit portions associated with the cMUT elements) becomes a top half and a bottom half. The cMUT elements overlap these two areas at the top edges and the bottom edges (as well as overlapping the edges into the skipped circuit portions).

The skipping of ASIC control circuit portions allows even greater flexibility in the range of frequencies and/or apertures that can be achieved. In this way, the same ASIC design can be reused for multiple transducer variants, which is very beneficial, as the ASIC design is the most expensive part of the entire transducer.

The invention is of general interest for the manufacture of 2D cMUT arrays that are monolithically connected to control circuits. The invention is particularly interesting to realize relatively large transducer arrays and modular arrays.

Various examples above enable modular transducer arrays to be formed, by compiling transducer arrays with flexible length by combining a number of smaller transducer modules. It also is possible to realize transducer arrays with different lengths on one wafer. For example, transducer arrays with a length of 1, 2, 3, 4, 5 or 6 cm can be realized by connecting modules with a length of 1 cm. It is attractive to realize small transducer modules to reduce IC foundry costs. Multiple design masks can be placed on a single reticle (a so-called multi layer reticle, MLR process). A modular transducer approach can also significantly reduce ASIC design time.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An ultrasound transducer, comprising:
a control circuit (202) which comprises an integrated circuit, the integrated circuit comprising an array of circuit portions (204);
an array of capacitive micromachined ultrasound transducer, cMUT, elements (304) provided to overlay the control circuit; and
an array of vias (205), with at least one via providing a coupling between each cMUT element and a respective one of the circuit portions said CMUT element is arranged to overlay, those circuit portions together defining a circuit area,
**characterized in that**
the pitch of the array of cMUT elements is greater than the pitch of the circuit portions in at least one direction of the array such that in said at least one direction, the cMUT elements extend beyond one or both sides of the circuit area in said at least one direction, wherein the at least one direction of the array comprises a row direction or both row and column directions.

2. The ultrasound transducer according to claim 1, wherein the cMUT elements (304) each occupy an area and there are spaces defined between the occupied areas, and wherein each via (205) is located at a respective one of the spaces.

3. The ultrasound transducer according to claim 1 or 2, wherein each via (205) connects to the circuit portion beneath at a relative position within the shape of the circuit portion, wherein there are at least two different relative positions such that a first cMUT element connects to its respective circuit portion at a first relative position, and a second cMUT element connects to its respective circuit portion at a second relative position.

4. The ultrasound transducer according to claim 3, wherein along each row of cMUT elements (304), each relative position is different in the row direction, and the cMUT elements at one end of each row overlap one side of the circuit area in the row direction.

5. The ultrasound transducer according to claim 4, wherein the cMUT elements (304) at a same end of each row overlap a same side of the circuit area.

6. The ultrasound transducer according to claim 4 or 5, wherein along each column of cMUT elements, each relative position is different in the column direction, and the cMUT elements at one end of each column overlap one side of the circuit area in the column direction.

7. The ultrasound transducer according to claim 6, wherein the cMUT elements at a same end of each column overlap a same side of the circuit area.

8. The ultrasound transducer according to any one of claims 4 to 7, wherein along each row of cMUT elements, the via of a first set of cMUT elements is located at a space in a first position relative to the cMUT element, and the via of a second set of cMUT elements is located at a second, different, position relative to the cMUT element, and wherein the cMUT elements at each end of each row overlap the respective side of the circuit area in the row direction.

9. The ultrasound transducer according to any one of claims 4 to 7, wherein the via (205) of each cMUT element is located at a space at a same position relative to the cMUT element.

10. The ultrasound transducer according to claim 4, wherein the cMUT elements are staggered between adjacent rows by half a cMUT element pitch.

11. The ultrasound transducer according to any one of claims 1 to 10, wherein the control circuit has more circuit portions than the number of cMUT elements.

12. The ultrasound transducer according to claim 11, wherein the cMUT elements extend beyond one side of the circuit area and the spare circuit portions are at an opposite side.

13. The ultrasound transducer according to claim 12, wherein the cMUT elements extend beyond two orthogonal sides of the circuit area and the spare circuit portions are at both opposite sides.

14. An ultrasound transducer arrangement, comprising:
a plurality of ultrasound transducers, at least some of which are according to any one of claims 1 to 13, forming a tiled arrangement,
wherein the control circuits are spaced apart and the cMUT elements of the ultrasound transducers together form a regular array.

15. An ultrasound transducer arrangement according to claim 14, wherein the cMUT elements at each end of each row overlap a same respective overlap row side of the circuit area in the row direction, and the ultrasound transducer arrangement comprises:
a row of two ultrasound transducers each according to any one of claims 1 to 13, with the overlap row sides facing each other; or
a row of three ultrasound transducers with the outer two each according to any one of claims 1 to 13, with the row overlap sides facing each other, and a further ultrasound transducer in the middle; or
a 2x2 array of ultrasound transducers each according to any one of claims 1 to 13 and wherein the cMUT elements at each end of each column overlap a same respective overlap column side of the circuit area in the column direction.

## Patentansprüche

1. Ultraschallwandler, umfassend:
eine Steuerschaltung (202), welche eine integrierte Schaltung umfasst, wobei die integrierte Schaltung ein Array von Schaltungsabschnitten (204) umfasst;
ein Array von kapazitiven mikrobearbeiteten Ultraschallwandler-, cMUT-Elementen (304), welche bereitgestellt sind, um die Steuerschaltung zu überlagern; und
ein Array von Durchkontaktierungen (205), wobei zumindest eine Durchkontaktierung eine Kopplung zwischen jedem cMUT-Element und einem jeweiligen der Schaltungsabschnitte bereitstellt, die zu überlagern das CMUT-Element angeordnet ist, wobei solche Schaltungsabschnitte zusammen einen Schaltungsbereich definieren,
**dadurch gekennzeichnet, dass**
der Abstand des Arrays von cMUT-Elementen größer als der Abstand der Schaltungsabschnitte in zumindest einer Richtung des Arrays ist, sodass sich die cMUT-Elemente in dieser zumindest einen Richtung über eine oder beide Seiten des Schaltungsbereichs in der zumindest einen Richtung hinaus erstrecken, wobei die zumindest eine Richtung des Arrays eine Zeilenrichtung oder beide, Zeilen- und Spaltenrichtungen umfasst.

2. Ultraschallwandler nach Anspruch 1, wobei die cMUT-Elemente (304) jeweils einen Bereich einnehmen und zwischen den eingenommenen Bereichen Zwischenräume definiert sind, und wobei sich jede Durchkontaktierung (205) an einem jeweiligen der Zwischenräume befindet.

3. Ultraschallwandler nach Anspruch 1 oder 2, wobei jede Durchkontaktierung (205) an einer relativen Position innerhalb der Form des Schaltungsabschnitts mit dem darunterliegenden Schaltungsabschnitt verbunden ist, wobei es zumindest zwei unterschiedliche relative Positionen gibt, sodass ein erstes cMUT-Element an einer ersten relativen Position mit seinem jeweiligen Schaltungsabschnitt verbunden ist, und ein zweites cMUT-Element an einer zweiten relativen Position mit seinem jeweiligen Schaltungsabschnitt verbunden ist.

4. Ultraschallwandler nach Anspruch 3, wobei entlang jeder Zeile von cMUT-Elementen (304) jede relative Position in Zeilenrichtung unterschiedlich ist, und die cMUT-Elemente an einem Ende jeder Zeile eine Seite des Schaltungsbereichs in Zeilenrichtung überlappen.

5. Ultraschallwandler nach Anspruch 4, wobei die cMUT-Elemente (304) an einem gleichen Ende jeder Zeile eine gleiche Seite des Schaltungsbereichs überlappen.

6. Ultraschallwandler nach Anspruch 4 oder 5, wobei entlang jeder Spalte von cMUT-Elementen jede relative Position in Spaltenrichtung unterschiedlich ist, und die cMUT-Elemente an einem Ende jeder Spalte eine Seite des Schaltungsbereichs in Spaltenrichtung überlappen.

7. Ultraschallwandler nach Anspruch 6, wobei die cMUT-Elemente an einem gleichen Ende jeder Spalte eine gleiche Seite des Schaltungsbereichs überlappen.

8. Ultraschallwandler nach einem der Ansprüche 4 bis 7, wobei sich entlang jeder Zeile von cMUT-Elementen die Durchkontaktierung eines ersten Satzes von cMUT-Elementen an einem Zwischenraum in einer ersten Position in Bezug zum cMUT-Element befindet, und sich die Durchkontaktierung eines zweiten Satzes von cMUT-Elementen an einer zweiten, unterschiedlichen Position in Bezug zum cMUT-Element befindet, und wobei die cMUT-Elemente an jedem Ende jeder Zeile die jeweilige Seite des Schaltungsbereichs in der Zeilenrichtung überlappen.

9. Ultraschallwandler nach einem der Ansprüche 4 bis 7, wobei sich die Durchkontaktierung (205) jedes cMUT-Elements in einem Zwischenraum an einer gleichen Position in Bezug zum cMUT-Element befindet.

10. Ultraschallwandler nach Anspruch 4, wobei die cMUT-Elemente zwischen benachbarten Zeilen um einen halben cMUT-Elementabstand versetzt sind.

11. Ultraschallwandler nach einem der Ansprüche 1 bis 10, wobei die Steuerschaltung mehr Schaltungsabschnitte aufweist als die Anzahl der cMUT-Elemente.

12. Ultraschallwandler nach Anspruch 11, wobei sich die cMUT-Elemente über eine Seite des Schaltungsbereichs hinaus erstrecken und die freien Schaltungsabschnitte auf einer gegenüberliegenden Seite liegen.

13. Ultraschallwandler nach Anspruch 12, wobei sich die cMUT-Elemente über zwei orthogonale Seiten des Schaltungsbereichs hinaus erstrecken und die freien Schaltungsabschnitte auf beiden gegenüberliegenden Seiten liegen.

14. Ultraschallwandleranordnung, umfassend:
eine Vielzahl von Ultraschallwandlern, wobei zumindest einige nach einem der Ansprüche 1 bis 13 sind, welche eine gekachelte Anordnung bilden,
wobei die Steuerschaltungen voneinander beabstandet sind und die cMUT-Elemente der Ultraschallwandler zusammen ein regelmäßiges Array bilden.

15. Ultraschallwandleranordnung nach Anspruch 14, wobei die cMUT-Elemente an jedem Ende jeder Zeile eine gleiche jeweilige Überlappungszeilenseite des Schaltungsbereichs in der Zeilenrichtung überlappen, und die Ultraschallwandleranordnung umfasst:
eine Zeile von jeweils zwei Ultraschallwandlern jeweils nach einem der Ansprüche 1 bis 13, wobei die Überlappungszeilenseiten einander zugewandt sind; oder
eine Zeile von drei Ultraschallwandlern, wobei die äußeren beiden nach einem der Ansprüche 1 bis 13 sind, wobei die Überlappungszeilenseiten einander zugewandt sind, und ein weiterer Ultraschallwandler in der Mitte; oder
ein 2x2-Array von Ultraschallwandlern, jeweils nach einem der Ansprüche 1 bis 13 und wobei die cMUT-Elemente an jedem Ende jeder Spalte jeweils eine gleiche Überlappungsspaltenseite des Schaltungsbereichs in der Spaltenrichtung überlappen.

## Revendications

1. Transducteur ultrasonore, comprenant :
un circuit de commande (202) qui comprend un circuit intégré, le circuit intégré comprenant un réseau de parties de circuit (204) ;
un réseau d'éléments de transducteur ultrasonore micro-usiné capacitif, cMUT, (304) prévu pour recouvrir le circuit de commande ; et
un réseau de trous d'interconnexion (205), avec au moins un trou d'interconnexion fournissant un couplage entre chaque élément de cMUT et l'une respective des parties de circuit que ledit élément de cMUT est conçu pour recouvrir, ces parties de circuit définissant ensemble une zone de circuit,
**caractérisé en ce que**
le pas du réseau d'éléments de cMUT est supérieur au pas des parties de circuit dans au moins une direction du réseau de telle sorte que dans ladite au moins une direction, les éléments de cMUT s'étendent au-delà d'un ou des deux côtés de la zone de circuit dans ladite au moins une direction, dans lequel la au moins une direction du réseau comprend une direction de ligne ou à la fois des directions de ligne et de colonne.

2. Transducteur ultrasonore selon la revendication 1, dans lequel les éléments de cMUT (304) occupent chacun une zone et il y a des espaces définis entre les zones occupées, et dans lequel chaque trou d'interconnexion (205) est situé au niveau d'un espace respectif des espaces.

3. Transducteur ultrasonore selon la revendication 1 ou 2, dans lequel chaque trou d'interconnexion (205) se connecte à la partie de circuit située en dessous à une position relative dans la forme de la partie de circuit, dans lequel il y a au moins deux positions relatives différentes de telle sorte qu'un premier élément de cMUT se connecte à sa partie de circuit respective à une première position relative, et un second élément de cMUT se connecte à sa partie de circuit respective à une seconde position relative.

4. Transducteur ultrasonore selon la revendication 3, dans lequel le long de chaque rangée d'éléments de cMUT (304), chaque position relative est différente dans la direction de rangée, et les éléments de cMUT à une extrémité de chaque rangée chevauchent un côté de la zone de circuit dans la direction de rangée.

5. Transducteur ultrasonore selon la revendication 4, dans lequel les éléments de cMUT (304) à une même extrémité de chaque rangée chevauchent un même côté de la zone de circuit.

6. Transducteur ultrasonore selon la revendication 4 ou 5, dans lequel le long de chaque colonne d'éléments de cMUT, chaque position relative est différente dans la direction de colonne, et les éléments de cMUT à une extrémité de chaque colonne chevauchent un côté de la zone de circuit dans la direction de colonne.

7. Transducteur ultrasonore selon la revendication 6, dans lequel les éléments de cMUT à une même extrémité de chaque colonne chevauchent un même côté de la zone de circuit.

8. Transducteur ultrasonore selon l'une quelconque des revendications 4 à 7, dans lequel le long de chaque rangée d'éléments de cMUT, le trou d'interconnexion d'un premier ensemble d'éléments de cMUT est situé dans un espace dans une première position par rapport à l'élément de cMUT, et le trou d'interconnexion d'un deuxième ensemble d'éléments de cMUT est situé à une deuxième position différente par rapport à l'élément de cMUT, et dans lequel les éléments de cMUT à chaque extrémité de chaque rangée chevauchent le côté respectif de la zone de circuit dans la direction de rangée.

9. Transducteur ultrasonore selon l'une quelconque des revendications 4 à 7, dans lequel le trou d'interconnexion (205) de chaque élément de cMUT est situé au niveau d'un espace à une même position par rapport à l'élément de cMUT.

10. Transducteur ultrasonore selon la revendication 4, dans lequel les éléments de cMUT sont décalés entre des rangées adjacentes d'un demi-pas d'élément de cMUT.

11. Transducteur ultrasonore selon l'une quelconque des revendications 1 à 10, dans lequel le circuit de commande présente plus de parties de circuit que le nombre d'éléments de cMUT.

12. Transducteur à ultrasons selon la revendication 11, dans lequel les éléments de cMUT s'étendent au-delà d'un côté de la zone de circuit et les parties de circuit de rechange se trouvent au niveau d'un côté opposé.

13. Transducteur ultrasonore selon la revendication 12, dans lequel les éléments de cMUT s'étendent au-delà de deux côtés orthogonaux de la zone de circuit et les parties de circuit de rechange se trouvent au niveau des deux côtés opposés.

14. Agencement de transducteur ultrasonore, comprenant :
une pluralité de transducteurs ultrasonores, dont au moins certains sont conformes à l'une quelconque des revendications 1 à 13, formant un agencement en mosaïque,
dans lequel les circuits de commande sont espacés et les éléments de cMUT des transducteurs ultrasonores forment ensemble un réseau régulier.

15. Agencement de transducteurs ultrasonores selon la revendication 14, dans lequel les éléments de cMUT à chaque extrémité de chaque rangée chevauchent un même côté de rangée de chevauchement respectif de la zone de circuit dans la direction de rangée, et l'agencement de transducteurs ultrasonores comprend :
une rangée de deux transducteurs ultrasonores chacun selon l'une quelconque des revendications 1 à 13, les côtés de rangée de chevauchement se faisant face ; ou
une rangée de trois transducteurs ultrasonores avec les deux transducteurs extérieurs chacun selon l'une quelconque des revendications 1 à 13, avec les côtés de chevauchement de rangée se faisant face, et un autre transducteur ultrasonore au milieu ; ou
un réseau 2x2 de transducteurs ultrasonores, chacun selon l'une quelconque des revendications 1 à 13 et dans lequel les éléments de cMUT à chaque extrémité de chaque colonne chevauchent un même côté de colonne de chevauchement respectif de la zone de circuit dans la direction de colonne.
